# FASCICULE DE BREVET EUROPEEN

(11) **EP 2 559 453 B1**
(45) Date de publication et mention de la délivrance du brevet: **16.07.2014**
(21) Numéro de dépôt: 12170609.7
(22) Date de dépôt: 01.06.2012
(51) Int. Cl.: A61N 1/05

(54) **Sonde de stimulation multizone d'une cavité gauche du coeur, implantable dans le réseau coronarien**
Leiter zur Implantation in die Herzkranzgefäße zur mehrzonigen Stimulation eines linken Herzraums
Lead implantable in the coronary vessels for multi-zone stimulation of a left heart chamber

(30) Priorité: 18.08.2011 FR 1157394
(43) Date de publication de la demande: 20.02.2013
(73) Titulaire: Sorin CRM SAS, 92140 Clamart (FR)
(72) Inventeur: Ollivier, Jean-François, 91190 Villiers Le Bacle (FR); Anselme, Frédéric, 76230 Bois-Guillaume (FR); Shan, Nicolas, 91260 Juvisy sur Orge (FR)
(74) Mandataire: Dupuis-Latour, Dominique

(56) Documents cités:
- WO-A2-2006/023930
- FR-A1- 2 801 509
- US-A- 5 423 772
- US-A- 5 925 073
- US-A1- 2003 208 220
- US-A1- 2003 220 677
- US-A1- 2004 064 024
- US-A1- 2009 299 447
- US-B1- 6 178 355

## Description

L'invention concerne les "dispositifs médicaux implantables actifs" tels que définis par la Directive 90/385/CEE du 20 juin 1990 du Conseil des communautés européennes, notamment les implants permettant de surveiller en continu le rythme cardiaque et délivrer si nécessaire au coeur des impulsions électriques de stimulation ou de resynchronisation.

Elle concerne plus précisément les sondes de stimulation cardiaque destinées à être implantées dans le réseau coronarien du coeur pour permettre la stimulation d'une cavité gauche ou droite, ventricule ou oreillette.

A la différence des cavités droites pour lesquelles il suffit d'implanter des sondes endocavitaires via le réseau veineux périphérique droit, la mise en place de sondes permanentes dans une cavité du coeur gauche impliquerait des risques opératoires importants, par exemple le risque de passage de bulles vers le réseau vasculaire cérébral situé en aval du ventricule gauche.

Une première technique, décrite par exemple par le US 2009/0299447 A1, consiste à appliquer une sonde épicardique contre la paroi extérieure du myocarde, face à la cavité à stimuler.

Mais pour stimuler une cavité gauche, la technique la plus souvent utilisée - et qui est celle à laquelle se réfère l'invention - consiste à introduire une sonde non pas dans la cavité à stimuler mais dans le réseau coronarien, la sonde étant pourvue d'une électrode qui sera appliquée contre la paroi de l'épicarde et orientée vers le ventricule gauche ou l'oreillette gauche, selon le cas. Ces sondes stimulent le muscle cardiaque via une ou plusieurs électrodes ponctuelles dont la position est fonction de la trajectoire prédéfinie de la veine canulée.

Une sonde de ce type est par exemple le modèle *Situs LV,* commercialisé par Sorin CRM (Clamart, France) et décrit dans le EP 0 993 840 A1 (ELA Medical).

Le US 2003/0220677 A1 décrit également une sonde de ce même type. L'introduction d'une telle sonde se fait par le sinus coronaire, depuis son débouché dans l'oreillette droite. La sonde est ensuite poussée et orientée le long du réseau des veines coronaires jusqu'au site choisi. Cette intervention est très délicate compte tenu des particularités du réseau veineux et de ses voies d'accès, avec notamment le passage de valvules et tortuosités ainsi que la diminution progressive de diamètre du conduit au fur et à mesure de la progression de la sonde dans la veine coronaire sélectionnée.

Une fois la veine cible atteinte, le chirurgien recherche un site de stimulation satisfaisant, avec un bon contact électrique de l'électrode de stimulation contre le tissu de l'épicarde, ce contact devant être maintenu malgré les variations ou sollicitations diverses au cours du temps.

Il a été proposé de disposer plusieurs électrodes le long du corps de sonde pour augmenter les chances d'un compromis acceptable, en donnant éventuellement au corps de sonde une conformation particulière. Le chirurgien peut ainsi sélectionner, parmi les diverses électrodes présentes sur le corps de sonde, celle procurant la meilleure efficacité sur le plan électrique et hémodynamique. Une telle sonde à électrodes multiples est décrite notamment dans le EP 1 938 861 A1 (ELA Medical).

Ces sondes permettent en particulier de mettre en oeuvre le concept de "repositionnement électronique", visant à diriger ou rediriger le champ électrique entre différentes électrodes disposées le long de la sonde de stimulation de la cavité gauche et/ou avec une des électrodes de la sonde de stimulation de la cavité droite. Cette technologie permet de gérer les micro-déplacements ou les évolutions du comportement hémodynamique (*reverse modeling*), simplement par reprogrammation du générateur par voie télémétrique à travers la peau, donc sans réintervention chirurgicale lourde.

La contrepartie de cette solution est une complexité grandissante de la structure de la sonde : la multiplication du nombre d'électrodes entraîne une multiplication du nombre de composants, et donc des liaisons électriques, ou nécessite le recours à des circuits de multiplexage pour la sélection des diverses électrodes présentes sur la sonde. Tout ceci conduit à un accroissement des risques mécaniques de rupture.

Le US 2009/157136 A1 décrit une technique de recherche d'un site de stimulation optimal au moyen d'un cathéter temporaire de *mapping* à introduire dans le sinus coronaire. Ce cathéter est soit un tube flexible ouvert à ses deux extrémités, soit un fil-guide. Dans l'un ou l'autre cas, il est doté en partie distale de multiples électrodes électriquement indépendantes, et en partie proximale d'un connecteur de liaison à un système d'acquisition qui permettra d'identifier le meilleur site de stimulation par un algorithme basé sur le mouvement cardiaque. Une sonde définitive multiélectrode permanente classique de diamètre standard 4,5 à 6 French (1,5 à 2 mm) est ensuite placée jusqu'à la position sélectionnée, via le guide (technique dite OTW, *Over The Wire* ou "filoguidage" classique), ou dans le tube du cathéter temporaire.

Une autre tendance des développements récents en matière de sondes de stimulation du ventricule gauche est la réduction du diamètre de la partie implantable dans le réseau coronaire, jusqu'à un diamètre de 4 French (1,33 mm). La taille du corps de sonde est en effet un facteur directement lié aux capacités de guidage contrôlé de la sonde dans le réseau coronaire veineux, de manière à pouvoir y sélectionner des sites de stimulation particuliers situés dans certaines veines collatérales. Ces sites sont atteints au moyen d'un cathéter de sous-sélection de veine servant à la mise en place d'un mandrin de guidage jusqu'au site choisi. Une fois la veine sélectionnée et le mandrin posé, le chirurgien fait progresser le corps de sonde qu'il glisse sur le mandrin, ce dernier jouant le rôle d'un fil support de faible diamètre guidant axialement le corps de sonde jusqu'à l'emplacement choisi (technique OTW de "filoguidage").

Ces diverses solutions présentent toutefois une double limitation, qui est celle :
- de la finesse de la sonde, dont le diamètre ne permet pas d'atteindre les veines collatérales les plus profondes : ainsi, pour la sonde *Situs* précitée, la sonde présente un diamètre de 6,6 French (2,2 mm) et nécessite un introducteur de 7 French (2,33 mm) de diamètre ; et
- du positionnement correct et du bon maintien en contact électrique de l'électrode contre le tissu à stimuler (paroi de l'épicarde).

Si les techniques précitées de sonde multi-électrodes et de repositionnement électronique permettent, plus ou moins bien, de s'affranchir de la seconde limitation, elles aggravent en revanche la première limitation, dans la mesure où la multiplication des électrodes et des composants ou conducteurs internes implique nécessairement un accroissement du diamètre du corps de sonde et diminue sa flexibilité, rendant difficile voire impossible le passage des tortuosités.

L'invention vise à s'affranchir de ces deux limitations, par une sonde de stimulation du ventricule gauche dont la partie active :
- présente un très faible diamètre, permettant d'exploiter toute la longueur de la veine et d'utiliser de façon optimale toutes les veines présentes dans la zone basale ;
- garantisse un contact électrique d'excellente qualité et durable avec les tissus à stimuler ;
- et, surtout, multiplie ou élargit les zones de stimulation, permettant ainsi (contrairement aux sondes traditionnelles) de stimuler simultanément plusieurs zones de l'épicarde en améliorant de ce fait les chances d'une resynchronisation optimale.

À ce dernier égard, on a en effet constaté que la multiplication des points de stimulation sur le ventricule gauche est un facteur permettant d'améliorer de façon substantielle la qualité de la resynchronisation.

Les études en cours montrent toutefois qu'il est très difficile d'implanter concurremment deux sondes dans le réseau coronaire veineux. Une alternative connue consiste à doter un corps de sonde de plusieurs électrodes, jusqu'à quatre dans certains modèles. Il s'agit toutefois de sondes de diamètre relativement important, de l'ordre de 4 French (1,33 mm), du fait de la complexité des composants et liaisons nécessaires à la sélection des électrodes pour le repositionnement électronique. De plus, la position relative des couples d'électrodes sélectionnés est très limitée, ces couples d'électrodes étant en outre nécessairement positionnés dans la même veine coronaire, généralement la veine postéro-latéraie.

L'un des buts principaux de l'invention est de proposer une nouvelle configuration de sonde de stimulation de très faible diamètre, implantable dans le réseau coronaire veineux, autorisant pour améliorer l'efficacité de la stimulation : (i) un élargissement de la zone stimulée avec en outre (ii) la possibilité de stimuler deux zones distantes via deux veines distinctes, tout en gardant la simplicité d'implantation d'une sonde unique.

Le but de l'invention est également de proposer une telle sonde qui soit de structure simple (donc peu coûteuse à fabriquer, et présentant une fiabilité maximale) et qui s'affranchisse des problèmes liés à la conception et à l'emploi des sondes à électrodes multiples, dont on a exposé plus haut la complexité structurelle et fonctionnelle.

Le point de départ de l'invention est la constatation de la présence très fréquente (typiquement 60 à 80 % de la population de patients) d'anastomoses distales dans le réseau veineux coronaire, c'est-à-dire qu'à l'extrémité de certaines veines existe un passage vers une autre veine, avec de ce fait possibilité de communication entre deux veines distinctes au niveau de l'anastomose, via leurs extrémités distales respectives.

L'idée de base de l'invention consiste à prévoir une sonde comportant un microcâble, c'est-à-dire un élément flexible de diamètre au plus égal à 2 French (0,66 mm), électriquement isolé à l'exception de zones ponctuellement dénudées formant des électrodes de stimulation toutes reliées électriquement ensemble, et de passer cet - unique - microcâble dans une première veine (veine "aller") puis par l'anastomose vers une deuxième veine (veine "retour") en remontant dans celle-ci.

Le microcâble de faible diamètre permettra de canuler des veines de très faible diamètre, non exploitées à ce jour du fait de la taille excessive des sondes coronaires permanentes.

La répartition des électrodes (les zones ponctuellement dénudées) sur le microcâble est telle que les électrodes sont groupées en deux ensembles distincts, l'un destiné à définir des sites de stimulation dans la veine aller et l'autre destiné à définir des sites de stimulation dans la veine retour. Ces deux ensembles d'électrodes sont séparés par une région isolée correspondant à la partie la plus distale de la veine aller, la région de l'anastomose et la partie la plus distale de la veine retour.

La configuration des électrodes du microcâble permet ainsi, avec une unique sonde, de stimuler concomitamment deux zones relativement distantes, car situées dans deux veines distinctes. L'éloignement de ces deux zones et la multiplication des points de stimulation dans chacune des zones procure un effet particulièrement bénéfique pour la resynchronisation du fonctionnement du coeur.

Des préformes du microcâble favoriseront le contact des électrodes avec les parois des veines, et par là même la performance électrique.

Le microcâble pourra en outre, comme on le verra plus bas, être posé par des techniques conventionnelles, bien connues des praticiens, ne nécessitant donc aucun apprentissage ou habileté technique supplémentaire.

Plus précisément, la technique consiste à canuler la veine aller de manière classique, en introduisant un fil-guide le long de celle-ci puis à travers l'anastomose et la veine retour. Un microcathéter est alors avancé sur le fil-guide jusqu'à l'extrémité de celui-ci. Le fil-guide est alors retiré et remplacé par le microcâble portant les deux zones stimulantes avec leurs électrodes (zones ponctuellement dénudées). Une fois le microcâble avancé jusqu'à sa position définitive, le microcathéter est alors rétracté, permettant de découvrir les deux zones stimulantes du microcâble, dont chacune est active dans l'une des deux veines respectives, aller et retour. Essentiellement, l'invention propose une sonde de stimulation destinée à être implantée dans une veine du réseau coronarien pour la stimulation d'une cavité gauche du coeur. Cette sonde comporte, de manière en elle-même connue, notamment d'après le US 2003/0220677 A1 précité, un élément flexible en matériau conducteur comportant à son extrémité distale une partie libre active comprenant une pluralité de zones dénudées distinctes, destinées à venir en contact avec la paroi d'une veine cible du réseau coronaire de manière à constituer ainsi un réseau d'électrodes de stimulation reliées électriquement ensemble. L'élément flexible comprend en outre, côté proximal, des moyens de couplage à un générateur de dispositif médical implantable actif tel qu'un stimulateur cardiaque ou resynchroniseur. La partie libre active comprend, au moins, une portion ondulée proximale et une portion ondulée distale séparées par une portion intermédiaire. Les portions ondulées proximale et distale sont élastiquement déformables entre un état ondulé libre et un état déployé, sous contrainte radiale.

De façon caractéristique de l'invention, l'élément flexible est un élément dépourvu de lumière interne de type microcâble de diamètre au plus égal à 2 French (0,66 mm), la longueur en direction axiale de chacune des portions ondulées est comprise entre 1 et 5 cm à l'état déployé, et la longueur en direction axiale de la portion intermédiaire est comprise entre 5 et 20 cm.

Selon diverses formes de réalisation avantageuses :
- la dimension hors-tout en direction radiale de chacune des portions ondulées est comprise entre 10 et 25 mm à l'état libre ;
- chacune des portions ondulées présente à l'état libre la forme d'une période de sinusoïde, avec une zone dénudée au sommet de chaque demi-période de sinusoïde et une zone dénudée à l'extrémité située côté distal de la période de sinusoïde ;
- la surface totale exposée des zones dénudées de la partie libre active du microcâble est d'au plus 10 mm² ;
- le microcâble est formé d'une pluralité de brins toronnés ensemble, dont au moins certains sont des brins incorporant une âme en matériau radio-opaque de type platine-iridium ou tantale enveloppée dans une gaine en matériau mécaniquement endurant de type NiTi ou inox, ou inversement ;
- la sonde comprend en outre un microcathéter creux en matériau déformable, avec une lumière centrale ouverte à ses deux extrémités et dans lequel le microcâble est disposé à coulissement en s'étendant sur toute la longueur du microcathéter et au-delà de l'extrémité distale de celui-ci, la partie du microcâble émergeant au-delà de l'extrémité distale du microcathéter formant ladite partie libre active ;
- le microcathéter creux peut porter côté distal au moins une électrode de stimulation bipolaire, non électriquement reliée au microcâble, l'électrode de stimulation bipolaire la plus distale du microcathéter creux étant distante de la zone dénudée la plus proximale du microcâble d'un intervalle compris entre 5 et 15 mm.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre de façon générale le myocarde, avec les principales veines du réseau coronaire dans lequel a été introduite une sonde selon l'invention, destinée à la stimulation du ventricule gauche.
La Figure 2 illustre la partie d'extrémité du microcâble selon l'invention permettant la stimulation multizone.
La Figure 3 illustre l'ensemble des éléments de la sonde de l'invention, dans un mode de réalisation destiné à une stimulation unipolaire.
Les Figures 4a et 4b illustrent deux variantes d'une sonde selon l'invention, dans un mode de réalisation destiné à une stimulation bipolaire.

On va maintenant décrire des exemples de réalisation de la sonde selon l'invention.

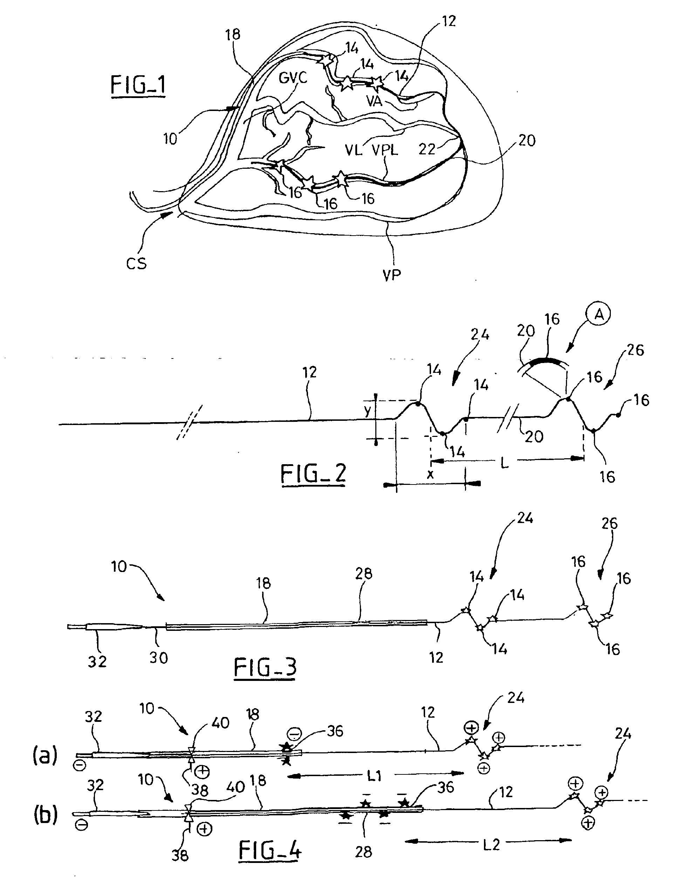

La Figure 1 illustre de façon générale le myocarde et les principaux vaisseaux du réseau coronaire, dans lequel on a introduit une sonde selon l'invention afin de stimuler le ventricule gauche.

Cette sonde est implantée par voie endocavitaire dans le réseau coronaire veineux via la veine cave supérieure, l'oreillette droite et l'entrée CS du sinus coronaire veineux. Le réseau coronaire veineux se développe ensuite en plusieurs branches à partir de la grande veine coronaire GVC, ces branches comprenant les veines postéro-latérale VPL, latérale VL, antéro-latérale VA et postérieure VP.

La référence 10 désigne de façon générale la sonde selon l'invention, qui comprend un microcâble 12 (dont la partie distale est représentée isolément Figure 2) introduit dans la veine antéro-latérale VA et qui porte une pluralité d'électrodes de stimulation 14 destinées à stimuler le ventricule gauche à partir de plusieurs sites situés dans cette veine VA. La sonde 10 comprend en outre, dans sa région proximale, un microcathéter 18 pénétrant dans le sinus coronaire et la grande veine cardiaque GVC jusqu'au débouché de la veine antéro-latérale VA.

De façon caractéristique, le microcâble 12 porte également, à distance des électrodes 14, une autre série d'électrodes 16 destinées à stimuler le ventricule gauche depuis une autre veine, par exemple la veine postéro-latérale VPL via une communication par une anastomose 22 reliant la veine antéro-latérale VA et la veine postéro-latérale VPL. Le microcâble traverse cette anastomose 22 et les régions les plus distales des deux veines VA et VPL le long d'une portion intermédiaire 20 dépourvue d'électrodes.

Grâce à cette configuration, il est possible non seulement de stimuler le ventricule gauche en plusieurs points de l'une des veines (du fait de la multiplication des électrodes 14 ou 16), mais en outre de prévoir deux zones relativement distantes de stimulation, respectivement la zone des électrodes 14 et la zone des électrodes 16, situées dans des régions proximales de deux veines différentes dans lesquelles il aurait été difficile de stabiliser ou fixer des sondes conventionnelles de stimulation du ventricule gauche, du fait du grand diamètre de l'embouchure de ces veines. Le microcâble 20 présente un diamètre au plus égal à 2 French (0,66 mm), typiquement de l'ordre de 0,5 à 2 French (0,16 à 0,66 mm).

Il est avantageusement réalisé à base de nitinol (alliage NiTi) ou d'acier type MP35NLT, matériaux dont l'avantage essentiel est leur extrême endurance en fatigue. Il s'agit d'obtenir nativement la résistance à la corrosion au niveau des électrodes, tout en garantissant la résistance à la fatigue impérativement requise.

Plus précisément, la structure du microcâble est une structure multifilaire dans laquelle chaque brin est constitué d'une âme en platine-iridium gainée par une épaisseur de nitinol ou d'acier MP35NLT - ou inversement, de manière à optimiser la réponse aux exigences à la fois de résistance à la corrosion et à la fatigue.

L'ensemble peut alors être revêtu :
- soit par une fine couche de parylène (par exemple de type C). Dans ce cas, des ouvertures plus ou moins complexes sont aménagées le long du microcâble, par exemple par ablation plasma, pour former les zones électriquement actives (électrodes) 14 et 16. Pour améliorer les performances électriques, ces zones peuvent être en outre revêtues par exemple de nitrure de titane ;
- soit par un tube polyuréthanne interrompu aux endroits des zones actives 14 et 16;
- soit par une ou plusieurs couches constituées de tubes en PET (polyéthylène téréphtalate), polymère fluoré, PMMA (polyméthacrylate de méthyle), PEEK (polyétheréthercétone), polyimide ou autre matériau similaire approprié.

Une telle structure de microcâble, sans lumière interne et avec plusieurs microfils tressés ensemble, est capable d'assurer à la fois l'endurance (à l'encontre des mouvements cardiaques) et la résistance aux sollicitations liées à la pose.

Ces types de microcâbles sont disponibles par exemple auprès de la société Fort Wayne Metals Inc., Fort Wayne, USA, et sont utilisés dans le domaine médical notamment pour réaliser des conducteurs de défibrillation - toutefois selon un arrangement de matériaux différent : dans ces applications connues, la structure est une structure multifilaire dans laquelle chaque brin inclut une âme en argent (pour améliorer la conductivité) gainée par une épaisseur d'inox ; ces microstructures, isolées ou non, sont ensuite incorporées dans un corps de sonde multi-lumière de construction classique.

Comme alternative, il est néanmoins possible de disposer un brin en platine-iridium au centre d'une structure multifilaire de type 1x7, le brin le plus fragile étant alors enlacé par les brins externes plus endurants.

Enfin, le platine-iridium peut être remplacé par tout matériau radio-opaque tel que le tantale.

Les parties dénudées 14 et 16 forment une succession d'électrodes individuelles, constituant ensemble un réseau d'électrodes reliées en série permettant de multiplier les points de contact avec la paroi de la veine et d'assurer ainsi une diffusion multi-zone de l'énergie de stimulation en plusieurs points du réseau coronaire et donc du ventricule gauche.

La surface individuelle de chaque électrode est d'au plus 1 mm², ce qui permet d'en disposer un nombre important sans dépasser une surface totale cumulée de 10 mm². Du fait de la faible surface active cumulée, on bénéficie ainsi des avantages d'une sonde dite à "haute densité de courant", en termes à la fois d'efficacité physiologique de la stimulation et de moindre consommation d'énergie - ceci tout en maximisant les chances de contact physique, donc électrique, des électrodes 14, 16 avec les tissus excitables, du fait de la multiplication de ces électrodes et de leur position au sommet des ondulations.

La Figure 2 illustre plus précisément la configuration de la partie distale du microcâble 12. Celle-ci comporte deux portions ondulées 24, 26 par exemple en forme d'une période de sinusoïde. Ces deux portions ondulées 24, 26 correspondent aux deux zones respectives de stimulation, à savoir la zone des électrodes 14 dans la veine antéro-latérale VA, d'une part, et la zone des électrodes 16 dans la veine postéro-latérale VPL, d'autre part.

On notera toutefois que, bien que dans cet exemple on ait représenté deux portions ondulées, il est possible d'en prévoir un nombre supérieur, par exemple si l'on souhaite stimuler l'une et/ou l'autre veine sur plusieurs zones, au lieu d'une seule zone par veine. De la même façon, l'exemple illustré qui montre trois électrodes pour chacune des portions ondulées, donc trois points de stimulation dans chaque zone, n'est aucunement limitatif, et il est possible de prévoir un nombre inférieur ou supérieur d'électrodes dans chacune de ces zones.

L'ondulation de chacune des portions 24, 26 est une préforme donnée au microcâble à l'état libre, avec par exemple une longueur x de la période de sinusoïde de l'ordre de 30 mm et une amplitude totale y en direction radiale de l'ordre de 10 à 25 mm.

Sur l'exemple décrit, les deux portions 24, 26 forment des ondulations s'étendant dans un plan commun, mais en variante il est possible de réaliser une structure dans laquelle les ondulations des portions 24, 26 s'étendent dans les trois dimensions de l'espace, chacune des portions 24, 26 s'étendant typiquement dans un cube enveloppe de 25 mm de côté environ.

Les deux portions ondulées 24, 26 sont séparées par une portion intermédiaire 20 dont la longueur L est comprise entre 5 et 15 cm.

Grâce à l'élasticité du microcâble, les préformes des portions ondulées 24, 26, sont déformables sous contrainte radiale lors du passage dans les veines du réseau coronaire, comme sur la configuration illustrée Figure 1. On notera que les préformes favorisent le contact des électrodes 14, 16 avec les tissus, et par là même la performance électrique de ces électrodes.

Avantageusement, des électrodes 14, 16 sont situées au sommet de chaque demi-période de la sinusoïde, comme cela est illustré plus précisément sur le détail repéré A sur la Figure 2.

On notera que la simplicité de la structure - microcâble isolé, avec des zones ponctuellement dénudées formant les électrodes - permet de localiser sans problème une électrode au sommet de l'ondulation, ce qui serait beaucoup plus délicat avec une structure de sonde coronaire conventionnelle. En effet, ces zones de courbure maximale sont a *priori* les plus sollicitées en fonctionnement, ce qui conduit généralement les fabricants des sondes conventionnelles à localiser les électrodes à mi-distance des sommets, bien que ces zones soient notablement moins favorables au contact avec la paroi de la veine.

De plus, cette localisation des parties dénudées offre la possibilité de sectoriser les électrodes, c'est-à-dire de faire en sorte que, vues en section droite, elles ne s'étendent pas sur toute la périphérie du microcâble, mais seulement sur un secteur angulaire situé du côté de la face extérieure de la courbure, c'est-à-dire tourné vers les tissus avec lesquels le contact s'opérera. Il est de ce fait possible de garder isolé l'intérieur de la courbure afin de limiter encore la surface stimulante, avec les avantages exposés plus haut.

Une électrode 14, 16 est également située à l'extrémité de la période de sinusoïde, côté distal. On pourrait également prévoir, de la même façon, une quatrième électrode, à l'extrémité située côté proximal de la période de sinusoïde.

Sur la Figure 3, on a représenté l'ensemble des éléments de la sonde, avec le microcâble 12 logé dans le microcathéter 18.

La partie proximale 30 du microcâble 12 est prolongée par un connecteur 32, par exemple de type IS-1, assemblé en usine.

On va maintenant exposer la technique d'implantation de la sonde selon l'invention.

Cette sonde est implantée par une technique conventionnelle de type OTW ou "filoguidage" au moyen d'un mandrin très fin formant fil-guide, pourvu à son extrémité distale d'une terminaison très souple pour ne pas être traumatique et permettre son introduction directe dans les vaisseaux du réseau coronaire sans risque de perforation.

Au préalable, le chirurgien dispose un cathéter principal lui permettant d'accéder au débouché du sinus coronaire, et un cathéter de sous-sélection permettant de choisir, sous amplificateur de brillance, le chemin du réseau veineux qui permettra d'atteindre la veine cible.

Dans la mesure où les performances de *tracking* du microcâble, c'est-à-dire ses performances insuffisantes de transmission du couple et de la poussée depuis l'extrémité proximale, sa trop grande souplesse, ne permettent pas une canulation directe des veines aller et retour, il est nécessaire de procéder à la mise en place préalable d'un fil-guide et du microcathéter 18.

A cet effet, le chirurgien introduit dans le cathéter de sous-sélection le fil-guide, qu'il pousse pour le faire progresser à nu dans le réseau veineux coronaire de façon à sélectionner telle ou telle veine collatérale, dans le cas présent la veine "aller" choisie (ici la veine antéro-latérale VA), puis l'anastomose 22 et enfin la veine "retour" choisie (ici la veine postéro-latérale VPL) en remontant celle-ci.

Le chirurgien enfile ensuite sur le fil-guide le microcathéter 18, qu'il fait alors glisser et progresser sur le fil-guide jusqu'à l'extrémité de ce dernier. Après retrait du fil-guide, il enfile le microcâble 12 dans le microcathéter 18 à partir de l'extrémité proximale de celui-ci, puis pousse le microcâble 12 sur toute la longueur du microcathéter. Ce dernier est ensuite reculé pour découvrir les portions ondulées 24 et 26 et donc les électrodes 14, 16 de la partie active, stimulante, du microcâble, pour obtenir la configuration illustrée Figure 1, avec les deux groupes d'électrodes 14 et 16 disposés aux sites de stimulation respectifs choisis.

Comme on peut le constater, ces étapes de canulation de veine sont couramment pratiqués par les spécialistes de cette technique de pose, de sorte que l'implantation d'une sonde selon l'invention ne nécessite aucune nouvelle technique opératoire ni habileté particulière.

On remarquera que la solution de l'invention permet un placement optimal du microcâble grâce à la combinaison fil-guide/microcathéter.

On notera en outre la simplicité et la robustesse de l'ensemble, malgré le très faible diamètre. En effet, la ligne de conduction ne comporte aucune liaison critique telle que soudure ou collage, cette ligne de conduction étant constituée d'un élément unique et robuste, à savoir le microcâble. Les Figures 4a et 4b illustrent deux variantes de l'invention, dans un mode de réalisation destiné à une stimulation bipolaire.

Sur ces figures, le microcathéter 18 porte également des électrodes 36, reliées individuellement ou collectivement à un conducteur spécifique 38, isolé du microcâble 12. Cette liaison est réalisée au moyen d'un dispositif de connexion 40 tel que celui décrit par exemple dans la demande de brevet européen EP 2011 0180909 du 12.09.2001, se prévalant de la priorité de la demande française 10 59847 du 29.11.2010, au nom de la Demanderesse, pour un *"Ensemble de stimulation*/*défibrillation du ventricule gauche par voie endocavitaire ou depuis une veine du réseau coronarien".* Cette demande, relevant donc de l'article 54(3) CBE, décrit un dispositif permettant de réaliser à la fois une prise de contact électrique sur un microcâble et une immobilisation mécanique de celui-ci par rapport à une gaine, tout en assurant la continuité électrique d'un autre conducteur s'étendant le long de la gaine et relié à une électrode portée par le corps de sonde.

Les électrodes additionnelles 36 peuvent être disposées soit sur la partie principale du microcathéter 18 (Figure 4a), soit sur la partie 34 distale de celui-ci (Figure 4b). Dans le premier cas, il est prévu un intervalle minimal *L₁* entre les électrodes 36 et les électrodes 24 de l'ordre de 5 à 10 cm, et dans le deuxième cas un intervalle minimal *L₂* de 5 à 15 cm.

Grâce à une telle configuration, il est possible de réaliser une stimulation bipolaire, c'est-à-dire entre, d'une part, la ou les électrodes 36 et, d'autre part, les électrodes 14 et 16. En l'absence d'électrode(s) 36, la stimulation est une stimulation monopolaire, c'est-à-dire entre, d'une part, le boîtier du générateur et, d'autre part, les électrodes 14 et 16.

Une autre variante (non représentée) de configuration bipolaire consiste à juxtaposer deux microcâbles immobilisés et isolés dans une gaine multi-lumière miniaturisée commune, les deux microcâbles étant connectés individuellement aux deux pôles du connecteur 32. Il est ainsi possible d'alterner la polarité des électrodes dans une même zone de stimulation (zone des électrodes 14 ou 16), par retrait sélectif de la gaine isolante sur une portion de la périphérie. Une variante quadripolaire sur le même principe est également envisageable.

## Revendications

1. Une sonde de stimulation, destinée à être implantée dans le réseau co-ronarien veineux pour la stimulation d'une cavité gauche du coeur,
cette sonde (10) comprenant un élément flexible (12) en matériau conducteur, comportant à son extrémité distale une partie libre active comprenant une pluralité de zones dénudées (14, 16) distinctes, destinées à venir en contact avec la paroi d'une veine cible du réseau coronarien de manière à constituer ainsi un réseau d'électrodes de stimulation reliées électriquement ensemble, l'élément flexible comprenant en outre, côté proximal, des moyens (32) de couplage à un générateur de dispositif médical implantable actif tel qu'un stimulateur cardiaque ou resynchroniseur,
sonde (10) dans laquelle :
- la partie libre active comprend, au moins, une portion ondulée proximale (24) et une portion ondulée distale (26) séparées par une portion intermédiaire (20) ;
- les portions ondulées proximale et distale sont élastiquement déformables entre un état ondulé libre et un état déployé, sous contrainte radiale ;
- l'élément flexible (12) est un élément dépourvu de lumière interne de type microcâble de diamètre au plus égal à 2 French (0,66 mm) ; et
- la longueur en direction axiale de chacune des portions ondulées (24, 26) est comprise entre 1 et 5 cm à l'état déployé, **caractérisée en ce que** la longueur en direction axiale de la portion intermédiaire est comprise entre 5 et 20 cm.

2. La sonde de la revendication 1, dans laquelle la dimension hors-tout en direction radiale de chacune des portions ondulées (24, 26) est comprise entre 10 et 25 mm à l'état libre.

3. La sonde de la revendication 1, dans laquelle chacune des portions ondulées (24, 26) présente à l'état libre la forme d'une période de sinusoïde.

4. La sonde de la revendication 3, comprenant une zone dénudée (14, 16) au sommet de chaque demi-période de sinusoïde.

5. La sonde de la revendication 3, comprenant une zone dénudée (14, 16) à l'extrémité située côté distal de la période de sinusoïde.

6. La sonde de la revendication 1, dans laquelle la surface totale exposée des zones dénudées de la partie libre active du microcâble est d'au plus 10 mm².

7. La sonde de la revendication 1, dans laquelle le microcâble est formé d'une pluralité de brins toronnés ensemble, dont au moins certains sont des brins incorporant une âme en matériau radio-opaque de type platine-iridium ou tantale enveloppée dans une gaine en matériau mécaniquement endurant de type NiTi ou inox, ou inversement.

8. La sonde de la revendication 1, comprenant en outre un microcathéter creux (18) en matériau déformable, avec une lumière centrale ouverte à ses deux extrémités et dans lequel le microcâble est disposé à coulissement en s'étendant sur toute la longueur du microcathéter et au delà de l'extrémité distale de celui-ci, la partie du microcâble émergeant au-delà de l'extrémité distale du microcathéter formant ladite partie libre active.

9. La sonde de la revendication 8, dans laquelle le microcathéter creux (18) porte côté distal au moins une électrode de stimulation bipolaire (36), non électriquement reliée au microcâble.

10. La sonde de la revendication 9, dans laquelle l'électrode de stimulation bipolaire la plus distale du microcathéter creux est distante de la zone dénudée la plus proximale du microcâble d'un intervalle (L₁, L₂) compris entre 5 et 15 mm.

## Patentansprüche

1. Stimulationssonde, die dazu bestimmt ist, im venösen koronaren Netz implantiert zu werden, um eine linke Herzkammer zu stimulieren,
wobei dies Sonde (10) ein flexibles Element (12) aus Leitermaterial umfasst, das an seinem distalen Ende einen aktiven freien Teil aufweist, der mehrere verschiedene abisolierte Zonen (14, 16) enthält, die dazu bestimmt sind, mit der Wand einer Zielvene des koronaren Netzes in der Weise in Kontakt zu gelangen, dass dadurch ein Netz von Stimulationselektroden gebildet wird, die elektrisch miteinander verbunden sind, wobei das flexible Element außerdem auf der proximalen Seite Mittel (32) für die Kopplung mit einem Generator einer aktiven implantierbaren medizinischen Vorrichtung wie etwa einem Herzstimulator oder Herzschrittmacher umfasst,
wobei in der Sonde (10):
- der aktive freie Teil wenigstens einen proximalen gewellten Abschnitt (24) und einem distalen gewellten Abschnitt (26), die durch einen Zwischenabschnitt (20) getrennt sind, umfasst;
- der proximale und der distale gewellte Abschnitt unter radialer Beanspruchung zwischen einem freien gewellten Zustand und einem entfalteten Zustand elastisch verformbar sind;
- das flexible Element (12) ein Element ohne Innenlangloch des Typs Mikrokabel mit einem Durchmesser, der höchstens gleich 2 French (0,66 mm) ist, ist; und
- die Länge in axialer Richtung jedes der gewellten Abschnitte (24, 26) im entfalteten Zustand im Bereich von 1 bis 5 cm liegt, **dadurch gekennzeichnet, dass** die Länge in axialer Richtung des Zwischenabschnitts im Bereich von 5 bis 20 cm liegt.

2. Sonde nach Anspruch 1, wobei die Abmessung über alles in radialer Richtung jedes der gewellten Abschnitte (24, 26) im freien Zustand im Bereich von 10 bis 25 mm liegt.

3. Sonde nach Anspruch 1, wobei jeder der gewellten Abschnitte (24, 26) im freien Zustand die Form einer Sinuswellenperiode aufweist.

4. Sonde nach Anspruch 3, die am Scheitel jeder Sinuswellen-Halbperiode eine abisolierte Zone (14, 16) aufweist.

5. Sonde nach Anspruch 3, die an dem Ende, das sich auf der distalen Seite der Sinuswellenperiode befindet, eine abisolierte Zone (14, 16) aufweist.

6. Sonde nach Anspruch 1, wobei die gesamte freiliegende Oberfläche der abisolierten Zonen des aktiven freien Teils des Mikrokabels höchstens 10 mm² beträgt.

7. Sonde nach Anspruch 1, wobei das Mikrokabel aus mehreren miteinander verdrillten Fasern gebildet ist, wovon wenigstens bestimmte Fasern Fasern sind, die einen Kern aus einem für Hochfrequenzen undurchlässigen Material des Typs Platin-Iridium oder Tantal, der in einen Mantel aus einem mechanisch beständigen Material des Typs NiTi oder Inox eingehüllt ist, oder umgekehrt, umfassen.

8. Sonde nach Anspruch 1, die außerdem einen hohlen Mikrokatheter (18) aus einem verformbaren Material mit einem an seinen zwei Enden offenen mittigen Langloch, in dem das Mikrokabel gleitend angeordnet ist, indem es sich über die gesamte Länge des Mikrokatheters und über sein distales Ende hinaus erstreckt, umfasst, wobei der Teil des Mikrokabels, der aus dem distalen Ende des Mikrokatheters austritt, den aktiven freien Teil bildet.

9. Sonde nach Anspruch 8, wobei der hohle Mikrokatheter (18) auf der distalen Seite wenigstens eine bipolare Stimulationselektrode (36) trägt, die mit dem Mikrokabel nicht elektrisch verbunden ist.

10. Sondern auch Anspruch 9, wobei die am meisten distale bipolare Stimulationselektrode des hohlen Mikrokatheters von der am meisten proximalen abisolierten Zone des Mikrokabels um ein Intervall (L₁, L₂) im Bereich von 5 bis 15 mm beabstandet ist.

## Claims

1. A pacing lead, intended to be implanted in the coronary venous system for the pacing of a left cavity of the heart,
this lead (10) comprising a flexible element (12) made of a conductive material, including at its distal end a free active part comprising a plurality of distinct stripped areas (14, 16), intended to come into contact with the wall of a target vein of the coronary system so as to form a network of pacing electrodes electrically connected to each other, the flexible element further comprising, on the proximal side, means (32) for the coupling to a generator of an active implantable medical device such as a pacemaker or a resynchronizer,
lead (10) wherein:
- the free active part comprises at least a proximal corrugated portion (24) and a distal corrugated portion (26) separated by an intermediate portion (20);
- the proximal and distal corrugated portions are elastically deformable between a free corrugated state and an expanded state, under a radial stress;
- the flexible element (12) is an element having no inner hole of the micro-cable type of a diameter at most equal to 2 French (0.66 mm); and
- the length in the axial direction of each of the corrugated portions (24, 26) is comprised between 1 and 5 cm in the expanded state, **characterized in that** the length in the axial direction of the intermediate portion is comprised between 5 and 20 cm.

2. The lead of claim 1, wherein the overall size in the radial direction of each of the corrugated portions (24, 26) is comprised between 10 and 25 mm in the free state.

3. The lead of claim 1, wherein each of the corrugated portions (24, 26) has in the free state the shape of a sinusoid period.

4. The lead of claim 3, comprising a stripped area (14, 16) at the apex of each sinusoid half-period.

5. The lead of claim 3, comprising a stripped area (14, 16) at the end located on the distal side of the sinusoid period.

6. The lead of claim 1, wherein the total exposed surface of the stripped areas of the free active part of the micro-cable is at most of 10 mm².

7. The lead of claim 1, wherein the micro-cable is formed of a plurality of threads stranded together, at least some of which are threads incorporating a core made of a radio-opaque material of the platinum-iridium or tantalum type, enveloped with a sheath made of a mechanically resistant material of the NiTi or stainless steel type, or the reverse.

8. The lead of claim 1, further comprising a hollow micro-catheter (18) made of a deformable material, with a central hole open at its two ends and in which the micro-cable is slidingly arranged, extending over the whole length of the micro-catheter and beyond the distal end the latter, wherein the part of the micro-cable that emerges beyond the distal end of the micro-catheter forms said free active part.

9. The lead of claim 8, wherein the hollow micro-catheter (18) carries on the distal side at least one bipolar pacing electrode (36), which is not electrically connected to the micro-cable.

10. The lead of claim 9, wherein the most distal bipolar pacing electrode relative to the hollow micro-catheter is separated from the more proximal stripped area relative to the micro-cable by an interval (L₁, L₂) comprised between 5 and 15 mm.
